# EUROPÄISCHE PATENTSCHRIFT

(11) **EP 3 318 223 B1**
(45) Veröffentlichungstag und Bekanntmachung des Hinweises auf die Patenterteilung: **23.09.2020**
(21) Anmeldenummer: 16196869.8
(22) Anmeldetag: 02.11.2016
(51) Int. Cl.: A61F 2/958, A61F 2/95

(54) **VORRICHTUNG ZUM EINBETTEN EINES AUF EINEM KATHETER ANGEORDNETEN BALLONS IN EIN IMPLANTAT UND ENTSPRECHENDES VERFAHREN**
DEVICE FOR EMBEDDING A BALLOON ARRANGED ON A CATHETER INTO AN IMPLANT AND CORRESPONDING METHOD
DISPOSITIF D'INTRODUCTION D'UN BALLON DISPOSÉ SUR UN CATHÉTER DANS UN IMPLANT ET PROCÉDÉ CORRESPONDANT

(43) Veröffentlichungstag der Anmeldung: 09.05.2018
(73) Patentinhaber: Biotronik AG, 8180 Bülach (CH)
(72) Erfinder: Hildebrand, Simon, 8180 Bülach (CH); Hämmerli, Alexandros, 8046 Zürich (CH)
(74) Vertreter: Galander, Marcus

(56) Entgegenhaltungen:
- WO-A1-2009/009636
- CH-A1- 705 977
- US-A- 6 063 092
- US-A1- 2011 213 451

## Beschreibung

Die Erfindung betrifft eine Vorrichtung zum Einbetten eines auf einem Katheter angeordneten Ballons in ein Implantat mit einem Heizelement sowie ein entsprechendes Verfahren.

Medizinische Implantate, insbesondere intraluminale Endoprothesen, für die unterschiedlichsten Anwendungen sind in großer Vielfalt aus dem Stand der Technik bekannt. Als Implantat im Sinne der vorliegenden Erfindung sind endovaskuläre Prothesen oder sonstige Endoprothesen, beispielsweise Stents (Gefäß-Stents (vaskuläre Stents, einschließlich Stents für die Anwendung im Bereich des Herzens und Herzklappen-Stents, zum Beispiel Mitralklappen-Stents, Pulmonalklappen-Stents) und Gallengang-Stents), Endoprothesen zum Verschließen von persistierenden Foramen Ovale (PFO), Stentgrafts zur Behandlung von Aneurysmen, Endoprothesen zum Verschließen eines ASG (Vorhofscheidewanddefekt, Atrioseptaldefekt) sowie Prothesen im Bereich des Hart- und Weichgewebes zu verstehen. Ein derartiges Implantat wird häufig mittels eines Katheters in das zu behandelnde Organ oder Gefäß eingesetzt.

Derartige Implantate weisen in vielen Fällen zumindest in einem Abschnitt eine durchbrochene hohlzylinderförmige (rohrförmige) und/oder hohlkegelförmige Struktur auf, die an beiden Längsenden offen ist. Die durchbrochene Struktur setzt sich häufig aus einer Vielzahl von Stegen zusammen, die miteinander verbunden und zwischen denen durchgehende Ausnehmungen (Zwischenräume) angeordnet sind.

Solche Implantate nehmen üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels des Katheters in das zu behandelnde Gefäß oder Organ durch enge Gefäße hindurch eingeführt und an der zu behandelnden Stelle positioniert werden. Hierbei ist das Implantat häufig auf einem ebenfalls komprimierten Ballon des Katheters, d. h. auf der Oberseite des Ballons, montiert. Am Ort der Behandlung wird das Implantat dann mittels des Ballons des Katheters dilatiert, um in den expandierten Zustand überführt zu werden. In dem expandierten Zustand verbleibt das Implantat in dem Gefäß oder Organ und ist dort festgelegt, nachdem der Katheter aus dem Körper des Behandelten entfernt wurde. Während des Transports des Implantats durch Organe und Gefäße des Körpers zu dem Ort der Behandlung im komprimierten Zustand ist es erforderlich, dass das Implantat fest auf dem Ballon sitzt und sicher den komprimierten Zustand einnimmt, sodass das Implantat zuverlässig zu dem Ort der Behandlung geführt werden kann, ohne die Gefäße und Organe, durch die es durchgeleitet wird, zu beschädigen. Ferner sollen eine Beschädigung der filigranen Struktur des Implantats oder des Ballons sowie Verunreinigungen des Implantats beziehungsweise des Ballons vermieden werden. Verunreinigungen können insbesondere deshalb entstehen, weil viele Implantate und/oder Ballons heutzutage Beschichtungen tragen, die ein therapeutisch wirksames Material (Medikament) enthalten.

Zur Anordnung des Implantats auf dem Ballon wird dieses auf den Ballon beispielsweise mittels Crimpen festgelegt bzw. montiert. Der zuverlässige Halt des Implantats auf dem Ballon wird jedoch erst durch einen Prozessschritt erreicht, der als Einbetten bezeichnet wird. Beim Einbetten wird das Ballonmaterial in die Zwischenräume des bereits montierten Implantats gepresst bzw. eingeprägt. Bei diesem Einbetten soll jedoch eine zu starke Inflation des Ballons beziehungsweise eine Expansion des Implantats verhindert werden.

Aus der Druckschrift US 2016/0096308 A1 ist für ein solches Einbetten eines Stents in einen Ballon ein Wärmetauscher bekannt, in dem ein Rohr angeordnet ist. Das Rohr dient zur Aufnahme einer Baugruppe bestehend aus Katheter mit Ballon und Stent. Diese Baugruppe wird nach der Anordnung der Baugruppe in dem Rohr in dem Wärmetauscher erwärmt und anschließend über den Katheter der Ballon aufgepumpt. Dann wird der Ballon bei einer zweiten Temperatur über eine vorgegebene Dauer gehalten und anschließend wieder abgekühlt.

Bei einem ähnlichen, aus der Druckschrift US 6,063,092 bekannten Verfahren wird eine Baugruppe aus einem Stent und einem darin angeordneten Katheter mit Ballon in einer mit einem longitudinalen Schlitz versehenen Teflon-Hülse angeordnet. Diese Hülse wird zudem in einer im Durchmesser reduzierbaren Hülle platziert, welche erwärmt werden kann. Zusammen mit dieser Hülle werden die Hülse und die Baugruppe in einem Heizblock angeordnet und erwärmt. Dann wird an dem Ballon ein Innendruck angelegt. Nach dem Abkühlen und dem Entfernen von Hülle und Hülse ist der Stent in dem Ballon eingebettet.

Die oben beschriebenen bekannten Verfahren haben den Nachteil, dass diese vergleichsweise lange Aufheiz- und Abkühlzeiten erfordern und aufgrund der notwendigen Montage der Hülse über der Baugruppe aufwendig sind. Zudem besteht bei Implantaten mit einer Medikament-Schicht die Gefahr von Cross-Kontamination.

Die Aufgabe der vorliegenden Erfindung besteht somit darin, eine Vorrichtung zu schaffen, die ein schnelles und somit kostengünstiges Einbetten eines Implantats in einen Ballon erlaubt. Ferner ist es die Aufgabe, ein entsprechendes Verfahren zum Einbetten anzugeben, das Cross-Kontamination vermeidet.

Die obige Aufgabe wird durch die Vorrichtung mit den Merkmalen des Anspruchs 1 oder 2 gelöst.

Die erfindungsgemäße Vorrichtung weist insbesondere eine Einbettform zur Anordnung in dem Heizelement auf, welche einen hohen Wärmefluss und eine zylindrische Ausnehmung, vorzugsweise eine Rohrgeometrie mit einer durchgehenden Ausnehmung, aufweist, die derart dimensioniert ist (insbesondere hinsichtlich Länge und Durchmesser), dass eine Baugruppe bestehend aus dem Katheter mit dem in das Implantat einzubettenden Ballon und dem auf der Außenseite des Ballons montierten, vorzugsweise aufgecrimpten, Implantat in die Ausnehmung der Einbettform einführbar und in der Ausnehmung anordnenbar ist, wobei eine Schutzfolie zwischen einer Innenwand der Ausnehmung der Einbettform und der Baugruppe vorgesehen ist.

Die Schutzfolie der erfindungsgemäßen Lösung erleichtert das Ein- und Ausführen der Baugruppe aus der Ausnehmung beziehungsweise in die Ausnehmung der Einbettform. Die geringe Stärke, das heißt die dünne Wand der Schutzfolie, bewirkt, dass die Wärmeisolation zwischen Heizelement und Baugruppe minimal ist, so dass eine schnelle Erwärmung bzw. Abkühlung der Baugruppe erfolgt. Hierdurch wird der gesamte Prozess beschleunigt und demzufolge werden Herstellungskosten gespart. Vorzugsweise hat die Schutzfolie eine Dicke von weniger als 25 µm, besonders bevorzugt von weniger als 20 µm. Die Dicke der Schutzfolie ist die kleinste Dimension der Abmessungen der Schutzfolie.

Erfindungsgemäß ist die Schutzfolie bandförmig gestaltet. Hierdurch kann die Baugruppe besonders leicht in die Einbettform ein- oder aus dieser ausgeführt werden. Alternativ kann die Schutzfolie als auf der Innenwand der Einbettform angeordnete Schicht gestaltet sein. Dies ist eine besonders kostengünstige Lösung für die Einbettform.

Das Ein- und Ausführen der Baugruppe sowie das Einbetten gestalten sich als besonders einfach und automatisierbar, wenn die Ausnehmung der Einbettform parallel zur Längsrichtung der Einbettform verläuft und durchgehend ausgebildet ist, wobei die bandförmige Schutzfolie lose an der Innenwand der Einbettform anliegt, das heißt nicht mit der Innenwand verbunden ist, und um die Baugruppe auf- und/oder abwickelbar ist sowie vorzugsweise an beiden Seiten in Längsrichtung der Ausnehmung über die durchgehende Ausnehmung der Einbettform hinaussteht. Vorzugsweise kann die bandförmige Schutzfolie von einer Rolle abgewickelt, durch die durchgehende Ausnehmung der Einbettform hindurchgeführt und nach Gebrauch auf eine Rolle aufgewickelt werden. Bei jeder Baugruppe, bei der das Implantat in den Ballon eingebettet werden soll, wird ein neuer Abschnitt der Schutzfolie verwendet, so dass jeder Abschnitt der Schutzfolie lediglich einmalig für das Einbetten eingesetzt wird. Hierdurch werden Verunreinigungen sowie mechanische Beschädigung der außen liegenden Elemente der Baugruppe vermieden.

Um zu gewährleisten, dass die Schutzfolie sicher die gesamte Baugruppe umgibt, ist die Breite der bandförmigen Schutzfolie größer als der Umfang der Ausnehmung der Einbettform. Hierdurch wird sichergestellt, dass die gesamte Innenwand der Einbettform abgedeckt ist. Die Breite der Schutzfolie wird senkrecht zu der Längsrichtung des Bandes gemessen. Beispielsweise beträgt die Breite der Schutzfolie mindestens 105% des Umfangs, vorzugsweise mindestens 110% des Umfangs der Ausnehmung der Einbettform. Besonders gute Eigenschaften hinsichtlich Ein- und Ausführen der Baugruppe in/aus der Ausnehmung der Einbettform werden gewährleistet, wenn die Schutzfolie aus einem Kunststoffmaterial, vorzugsweise mindestens einem Material der Gruppe umfassend Fluorpolymere (z. B. Polytetrafluorethylen, Perfluoralkoxy-Polymere) und Poleolefine (z. B. Polyethylen, Polypropylen) besteht.

Es wurde oben bereits erläutert, dass es von Vorteil ist, wenn die Einbettform einen hohen Wärmefluss besitzt. Diese Anforderung lässt sich in erster Linie durch dünnwandige Geometrie (geringe Masse) und durch ein Material mit einem hohen Wärmeleitkoeffizienten, vorzugsweise mit einem Wärmeleitkoeffizienten von über 10 W/(m^{∗}K), erreichen. Aufgrund der hohen Festigkeit metallischer Werkstoffe können metallische Einbettformen besonders dünnwandig auslegt werden, sodass ein metallischer Werkstoff für die Einbettform bevorzugt ist. Es kommt beispielsweise eine Wandstärke kleiner als 0,2 mm für Aluminiumlegierungen oder kleiner als 0,1 mm für Edelstahl (z. B. 1.4310) infrage. Zudem bieten metallische Werkstoffe hohe Wärmeleitkoeffizienten von über 10 W/(m^{∗}K) wodurch schnelle Aufheiz- und Abkühlzeiten und damit minimale Prozesszeiten ermöglicht werden. Beispielsweise können Edelstahl (z. B. 1.4301, 1.4310), Aluminium oder eine Aluminiumlegierung als ein Werkstoff für die Einbettform verwendet werden.

Für ein schnelles Abkühlen und somit eine verringerte Prozesszeit wird unterhalb des Heizelements ein Kühlelement angeordnet, das an seiner Oberseite mindestens eine Ausnehmung für die Durchleitung eines Kühlfluids (z. B. Wasser oder ein Gas, z. B. in der Form von gekühltem Stickstoff) aufweist. Das Heizelement kann vorzugsweise zweiteilig ausgebildet sein, wobei sich die beiden Teile zum Heizen der Baugruppe zusammenführen und um die Einbettform schließen lassen. Nach dem Einbetten können die beiden Teile des Heizelements wieder geöffnet werden, so dass die Einbettform mit der Baugruppe für das Kühlfluid zugänglich ist.

Die obige Erfindung wird ferner durch Verfahren zum Einbetten eines auf einem Katheter angeordneten Ballons in ein eine durchbrochene Struktur aufweisendes Implantat mit den folgenden Schritten gelöst:
- Herstellung einer Baugruppe bestehend aus einem Katheter mit Ballon und dem auf der Außenseite des Ballons montierten Implantat, vorzugsweise durch Aufcrimpen des Implantats auf den Ballon, insbesondere auf einen mittigen Abschnitt des Ballons,
- Einführen der Baugruppe in eine Ausnehmung einer Einbettform, die an ihrer Innenwand mit einer Schutzfolie ausgekleidet ist,
- Verbinden des Katheters mit einer Fluidquelle,
- Erwärmen der Einbettform mit Baugruppe in einem Heizelement über eine vorgegebene Zeit,
- Aufbringen eines vorgegebenen ersten Innendrucks an den Ballon mittels der mit dem Katheter verbundenen Fluidquelle,
- Abkühlen der Einbettform mit der Baugruppe bei vorgegebenem zweiten Innendruck in dem Ballon, der mittels der Fluidquelle aufgebracht wird,
- Deflation (Entlüften) des Ballons nach Abkühlen der Einbettform mit der Baugruppe,
- Entnehmen der Baugruppe aus der Einbettform.

Das erfindungsgemäße Verfahren erlaubt ein schnelles und kostengünstiges Einbetten des Implantats in den Ballon.

Es ist weiter von Vorteil, wenn vor dem Einführen der Baugruppe in die Ausnehmung der Einbettform eine bandförmige Schutzfolie um die Baugruppe gewickelt wird. Hierdurch wird das Einführen der Baugruppe in die Ausnehmung der Einbettform erleichtert.

Das Herausführen wird erleichtert und mechanische Beschädigung bzw. Cross-Kontamination reduziert, wenn beim Einführen der Baugruppe in die Einbettform und/oder Entnehmen der Baugruppe aus der Einbettform die Baugruppe zusammen/gemeinsam mit der bandförmigen Schutzfolie in die Ausnehmung der Einbettform hineingeführt beziehungsweise aus der Ausnehmung der Einbettform herausgeführt wird. Dies bedeutet, dass jeweils die Relativgeschwindigkeit zwischen Baugruppe und Schutzfolie Null beträgt, das heißt, dass sich Baugruppe und Schutzfolie jeweils beim Hineinführen und/oder Herausführen gleich schnell bewegen. Weiter bevorzugt wird nach dem Entnehmen die Schutzfolie von der Baugruppe abgewickelt.

Wie oben bereits beschrieben wurde, wird das Einbettverfahren dadurch erleichtert, dass die bandförmige Schutzfolie von einer Rolle abgewickelt, durch die Ausnehmung der Einbettform hindurchgeführt wird sowie vorzugsweise nach Verwendung für eine einzige Baugruppe wieder auf eine weitere Rolle aufgewickelt wird.

Weitere Ziele, Merkmale, Vorteile und Anwendungsmöglichkeiten der Erfindung ergeben sich aus der nachfolgenden Beschreibung eines Ausführungsbeispiels der erfindungsgemäßen Vorrichtung bzw. des erfindungsgemäßen Verfahrens anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch
- Fig. 1: eine erfindungsgemäße Vorrichtung in einer perspektivischen Ansicht von der Seite,
- Fig. 2: die Einbettform mit Schutzfolie der Vorrichtung gemäß Fig. 1 in einer perspektivischen Ansicht von der Seite,
- Fig. 3a: einen ersten Schritt eines Ausführungsbeispiels eines erfindungsgemäßen Verfahrens anhand eines Längsschnitts durch Einbettform und Schutzfolie,
- Fig. 3b: einen zweiten Schritt des Verfahrens gemäß Fig. 3a mit einem Längsschnitt durch eine Baugruppe und
- Fig. 3c: einen dritten Schritt des Verfahrens gemäß Fig. 3a mit einem Längsschnitt durch eine Baugruppe.

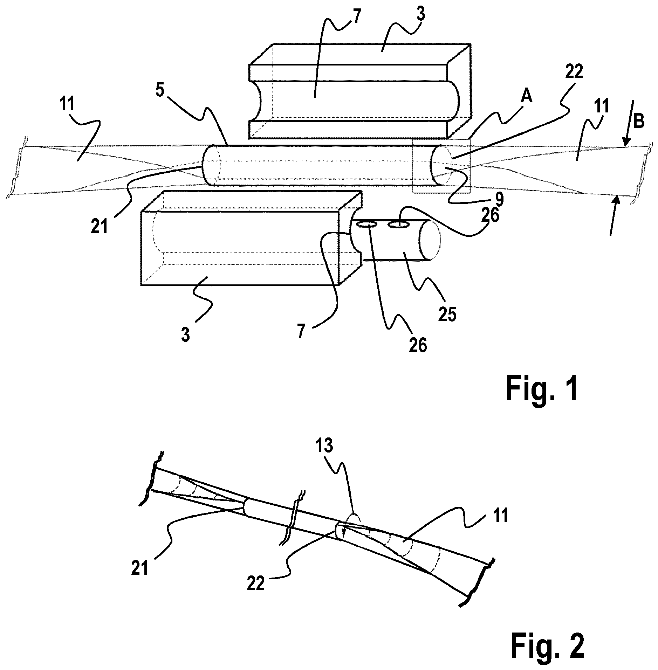

Die in Fig. 1 gezeigte erfindungsgemäße Vorrichtung zum Einbetten eines Ballons 30 eines Katheters 29 in ein durchbrochenes Implantat, beispielsweise einen Stent 31, weist ein zweiteiliges Heizelement 3 auf, in dem eine hohlzylindrisch ausgebildete Einbettform 5 angeordnet wird. Hierfür sind in jedem Teil des Heizelements 3 entsprechende Ausnehmungen 7 vorgesehen. Das Heizelement 3 ist dazu eingerichtet, dass es auf die Einbettform 5 Wärme übertragen kann, beispielsweise mittels Strahlung, Konvektion oder in Kontakt. Hierfür kann das Heizelement 3 beispielsweise als Widerstandsheizung, Induktionsheizelement, kapazitives Heizelement oder als Heizelement, das mit Infrarotstrahlung arbeitet, ausgestaltet sein.

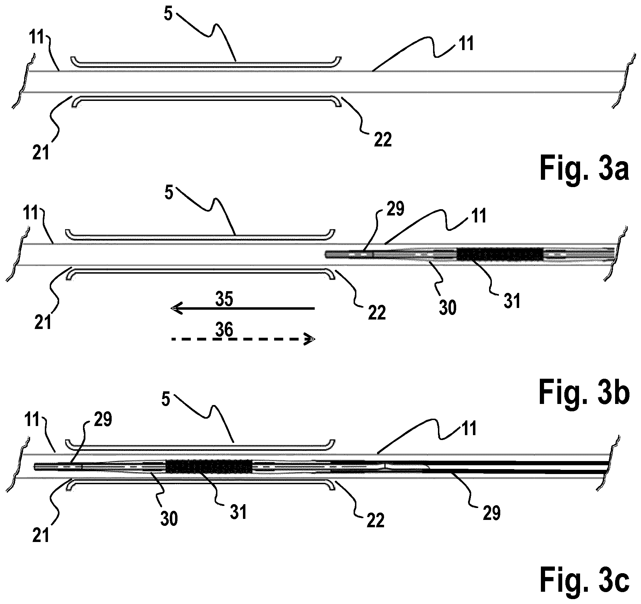

Zum Einbetten eines auf einem Katheter 29 angeordneten Ballons 30 in ein Implantat in der Form eines Stents 31, der zumindest abschnittsweise eine durchbrochene Struktur (Zwischenräume) aufweist, wird eine Baugruppe bestehend aus dem auf den Ballon 30 montierten Stent 31 und dem Ballon 30 mit Katheter 29 (siehe Fig. 3b und 3c) in die durchgehende Ausnehmung 9 der Einbettform 5 eingeführt. Um eine gute Wärmeübertragung von dem Heizelement 3 auf die Einbettform 5 zu erreichen, besteht diese aus einer dünnwandigen Geometrie (siehe oben) und einem Material mit einem hohen Wärmeleitkoeffizienten, beispielsweise einem metallischen Material (z. B. Edelstahl, eine Aluminiumlegierung oder Aluminium) oder sehr dünnwandigem Glas.

Durch die Einbettform 5 ist, wie in Fig. 3a gezeigt wird, eine bandförmige Schutzfolie 11 hindurchgeführt, welche zum Beispiel von einer Rolle abgewickelt (nicht dargestellt) und nach der Durchführung durch die Einbettform 5 wieder auf eine Rolle aufgewickelt werden kann (nicht dargestellt). Die Schutzfolie 11 ist innerhalb der durchgehenden Ausnehmung 9 der Einbettform 5 mittels einer Twistbewegung (siehe Pfeil 13 in Fig. 2 und Ausschnitt A in Fig. 1) derart umgelegt, dass die Schutzfolie 11 - im Querschnitt gesehen - gewissermaßen ein überlappendes "O" ausbildet, das innen an der Innenwand der durchgehenden Ausnehmung 9 der Einbettform 5 und außen an der Baugruppe anliegt. Hierfür weist die Schutzfolie 11, die vorzugsweise aus einem PTFE-Material besteht, eine Breite B auf, die größer ist als der Umfang der Einbettform 5 entlang ihrer Innenwand.

Die Baugruppe wird, wie in Fig. 3b dargestellt wird, vorzugsweise vor einem Ende 22 der Einbettform 5 in das durch Umlegen der Schutzfolie 11 ausgebildete innere Volumen der Schutzfolie 11 eingelegt und dann zusammen mit der Schutzfolie 11 durch Hindurchziehen der Schutzfolie 11 durch die Ausnehmung 9 der Einbettform 5 in die Einbettform 5 eingeführt. Die Richtung der gemeinsamen Bewegung von Baugruppe und Schutzfolie 11 wird in Fig. 3b durch den Pfeil 35 gekennzeichnet. Zuvor wurde die Baugruppe bestehend aus dem Katheter 29 mit Ballon 30 und dem auf der Außenseite des Ballons montierten Implantat, beispielsweise ein Stent 31, durch Aufcrimpen des Stents 31 auf einen mittig angeordneten Bereich des Ballons 30 gefertigt.

Durch das Auskleiden der durchgehenden Ausnehmung 9 mittels Schutzfolie 11 kommt weder der Ballon 30 noch der Stent 31 in Kontakt mit dem Material der Einbettform 5, sodass dort kein mechanischer Abrieb, das heißt keine Reib- und/oder Scherkräfte zwischen Baugruppe und Einbettform 5, entstehen. Nach dem vollständigen Einführen der Baugruppe in die durchgehende Ausnehmung 9 der Einbettform 5 (Position wie Fig. 3c gezeigt) wird jedes Teil des zweiteiligen Heizelements 3 auf die Einbettform 5 zu bewegt und umschließt diese vorzugsweise vollständig. Gleichzeitig wird der Katheter 29 mit einer Fluidquelle verbunden. Nun wird die in der Einbettform 5 angeordnete Baugruppe mittels des Heizelements 3 auf eine vorgegebene Temperatur, welche üblicherweise über der Glasübergangstemperatur des Ballonmaterials liegt (ca. 55°C bis 150°C), erwärmt. Danach wird mittels der mit dem Katheter 29 verbundenen Fluidquelle der Innendruck in dem Ballon 30 erhöht (ca. 5 bis 30 bar), sodass dieser geringfügig expandiert. Hierdurch wird das Ballonmaterial in die Zwischenräume des Stents 31 hineingepresst und somit in den Stent 31 eingebettet. Bei kontinuierlich aufgebrachtem Innendruck werden anschließend beide Teile des Heizelements 3 wieder von der Einbettform 5 entfernt und die Einbettform 5 kann abkühlen. Der Abkühlprozess wird durch ein unter der Einbettform 5 bzw. dem Heizelement 3 angeordnetes Kühlelement 25 unterstützt, durch dessen Rohr und auf der Oberseite angeordneten Öffnungen 26 ein Kühlfluid, beispielsweise Luft, zu der Einbettform 5 geleitet wird. Hierdurch kann eine schnellere Abkühlung der Einbettform 5 mit Baugruppe erreicht werden.

Nach ausreichender Abkühlung, das heißt üblicherweise unterhalb der Glasübergangstemperatur des Ballonmaterials (z. B. unterhalb von 55°C) wird der Druck im Katheter 29 durch die Fluidquelle wieder auf Umgebungsdruck reduziert und die Baugruppe zusammen mit der Schutzfolie 11 aus der Einbettform 5 herausgezogen. Die Richtung der gemeinsamen Bewegung von Schutzfolie 11 und Baugruppe wird in Fig. 3b mit dem Pfeil 36 dargestellt. Anschließend wird automatisch für den nächsten Einbettvorgang ein neuer Abschnitt der Schutzfolie 11 in der Einbettform 5 platziert. Hierfür kann die Schutzfolie beispielsweise mittels Hindurchziehen durch die Einbettform 5 in Richtung des Pfeils 36 (siehe Fig. 3b) weiterbewegt werden (sogenannte Leerfahrt). Hierdurch wird Cross-Kontamination, beispielsweise durch auf die Oberfläche des Stents aufgebrachte Medikamente, vermieden.

Außerhalb der Einbettform 5 kann die bandförmige Schutzfolie 11 so auseinander geschlagen werden, dass das Herausnehmen der Baugruppe mit dem in dem Stent 31 eingebetteten Ballon 30 leicht durchführbar ist. Hierdurch wird mechanischer Abrieb (das heißt Reib- und/oder Scherbelastungen) an der Baugruppe, insbesondere an dem Stent 31, vermieden.

Wie bereits oben erläutert wurde, wird die Schutzfolie 11 durch die durchgehende Ausnehmung 9 der Einbettform 5 hindurch für den nächsten Einbettvorgang weiter bewegt, sodass ein neuer Abschnitt der Schutzfolie 11 für den nächsten Einbettvorgang genutzt wird. Die verbrauchte Schutzfolie wird nach der Durchführung durch die Ausnehmung 9 der Einbettform 5 beispielsweise wieder auf eine Rolle aufgewickelt.

Die Schutzfolie 11 kann beispielsweise aus einem Kunststoffmaterial bestehen, wie beispielsweise PTFE, PFA, PE, PP, PA etc.. Insbesondere Fluorpolymere weisen hinsichtlich Temperaturbeständigkeit und Reibungskoeffizient geeignete Eigenschaften auf.

Die oben beschriebene erfindungsgemäße Vorrichtung und das erfindungsgemäße Verfahren erlaubt ein schnelles und kostengünstiges Einbetten eines Stents 31 in einen Ballon 30.

### Bezugszeichenliste:

- 3: Heizelement
- 5: Einbettform
- 7: Ausnehmung
- 9: Ausnehmung
- 11: Schutzfolie
- 13: Pfeil
- 21: erstes Ende der Einbettform 5
- 22: zweites Ende der Einbettform 5
- 25: Kühlelement
- 26: Öffnung
- 30: Ballon
- 31: Stent
- 29: Katheter
- 35: Pfeil
- 36: Pfeil
- A: Ausschnitt aus Fig. 1
- B: Breite der Schutzfolie 11

## Patentansprüche

1. Vorrichtung zum Einbetten eines auf einem Katheter (29) angeordneten Ballons (30) in ein eine durchbrochene Struktur aufweisendes Implantat (31),
- mit einem Heizelement (3) und
- mit einer Einbettform (5) zur Anordnung in dem Heizelement (3),
wobei die Einbettform (5) eine zylindrische Ausnehmung (9) aufweist, die derart dimensioniert ist, dass eine Baugruppe bestehend aus dem Katheter (29) mit Ballon (30) und dem auf der Außenseite des Ballons (30) montierten Implantat (31) in der Ausnehmung (9) der Einbettform (5) anordnenbar ist,
wobei eine Schutzfolie (11) zwischen einer Innenwand der Ausnehmung (9) der Einbettform (5) und der Baugruppe vorgesehen ist **dadurch gekennzeichnet, dass** die Schutzfolie (11) bandförmig und dass die Breite (B) der bandförmigen Schutzfolie größer ist als der Umfang der Ausnehmung (9) der Einbettform (5).

2. Vorrichtung zum Einbetten eines auf einem Katheter (29) angeordneten Ballons (30) in ein eine durchbrochene Struktur aufweisendes Implantat (31),
- mit einem Heizelement (3) und
- mit einer Einbettform (5) zur Anordnung in dem Heizelement (3),
wobei die Einbettform (5) eine zylindrische Ausnehmung (9) aufweist, die derart dimensioniert ist, dass eine Baugruppe bestehend aus dem Katheter (29) mit Ballon (30) und dem auf der Außenseite des Ballons (30) montierten Implantat (31) in der Ausnehmung (9) der Einbettform (5) anordnenbar ist,
wobei eine Schutzfolie (11) zwischen einer Innenwand der Ausnehmung (9) der Einbettform (5) und der Baugruppe vorgesehen ist, **dadurch gekennzeichnet, dass** die Schutzfolie (11) als mit der Innenwand der Einbettform (5) verbundene Schicht gestaltet ist.

3. Vorrichtung nach Anspruch 1, **dadurch gekennzeichnet, dass** die Ausnehmung (9) der Einbettform (5) parallel zur Längsrichtung der Einbettform (5) verläuft und durchgehend ausgebildet ist, wobei die bandförmige Schutzfolie (11) lose an der Innenwand der Einbettform (5) anliegt und um die Baugruppe auf- und/oder abwickelbar ist sowie vorzugsweise an beiden Seiten (21, 22) in Längsrichtung der Ausnehmung (9) über die durchgehende Ausnehmung (9) der Einbettform (5) hinaussteht.

4. Vorrichtung nach einem der vorhergehenden Ansprüche, **dadurch gekennzeichnet, dass** die Schutzfolie (11) aus einem Kunststoffmaterial, vorzugsweise mindestens einem Material der Gruppe umfassend PTFE, PFA, PE und LDPE, besteht.

5. Vorrichtung nach einem der vorhergehenden Ansprüche, **dadurch gekennzeichnet, dass** die Einbettform (5) einen hohen Wärmefluss besitzt.

6. Vorrichtung nach einem der vorhergehenden Ansprüche, **dadurch gekennzeichnet, dass** unterhalb des Heizelements (5) ein Kühlelement (25) angeordnet ist, das an seiner Oberseite mindestens eine Öffnung (26) für die Durchleitung eines Kühlfluids aufweist.

7. Verfahren zum Einbetten eines auf einem Katheter (29) angeordneten Ballons (30) in ein eine durchbrochene Struktur aufweisendes Implantat (31) mit den folgenden Schritten:
- Herstellung einer Baugruppe bestehend aus dem Katheter (29) mit Ballon (30) und dem auf die Außenseite des Ballons (30) montierten Implantat (31), vorzugsweise durch Aufcrimpen des Implantats (31) auf den Ballon (30),
- Einführen der Baugruppe in eine Ausnehmung (9) einer Einbettform (5), die an ihrer Innenwand mit einer Schutzfolie (11) ausgekleidet ist,
- Verbinden des Katheters (29) mit einer Fluidquelle,
- Erwärmen der Einbettform (5) mit Baugruppe in einem Heizelement (3) über eine vorgegebene Zeit,
- Aufbringen eines vorgegebenen ersten Innendrucks an den Ballon (30) mittels der mit dem Katheter (29) verbundenen Fluidquelle,
- Abkühlen der Einbettform (5) mit Baugruppe bei vorgegebenem zweiten Innendruck in dem Ballon (30),
- nach Abkühlen der Einbettform (5) mit der Baugruppe und Deflation des Ballons (30),
- Entnehmen der Baugruppe aus der Einbettform (5).

8. Verfahren nach Anspruch 7, **dadurch gekennzeichnet, dass** vor dem Einführen der Baugruppe in die Ausnehmung (9) der Einbettform (5) eine bandförmige Schutzfolie (11) um die Baugruppe gewickelt wird.

9. Verfahren nach einem der Ansprüche 7 bis 8, **dadurch gekennzeichnet, dass** beim Einführen der Baugruppe in die Einbettform (5) und/oder beim Entnehmen der Baugruppe aus der Einbettform (5) die Baugruppe zusammen mit der bandförmigen Schutzfolie (11) in die Ausnehmung (9) der Einbettform hineingeführt beziehungsweise aus der Ausnehmung (9) der Einbettform (5) herausgeführt wird, wobei vorzugsweise nach dem Entnehmen der Baugruppe anschließend die Schutzfolie (11) von der Baugruppe (5) abgewickelt wird.

10. Verfahren nach einem der Ansprüche 7 bis 9, **dadurch gekennzeichnet, dass** die bandförmige Schutzfolie (11) von einer Rolle abgewickelt und durch die Ausnehmung der Einbettform hindurchgeführt wird.

## Claims

1. A device for embedding a balloon (30) arranged on a catheter (29) in an implant (31) having an open-work structure, said device
- comprising a heating element (3) and
- comprising an embedding mould (5) for arrangement in the heating element (3),
wherein the embedding mould (5) has a cylindrical cavity (9), which is dimensioned in such a way that an assembly consisting of the catheter (29) with balloon (30) and of the implant (31) mounted on the outer side of the balloon (30) can be arranged in the cavity (9) of the embedding mould (5),
wherein a protective film (11) is provided between an inner wall of the cavity (9) of the embedding mould (5) and the assembly, **characterised in that** the width (B) of the strip-like protective film is greater than the circumference of the cavity (9) of the embedding mould (5).

2. A device for embedding a balloon (30) arranged on a catheter (29) in an implant (31) having an open-work structure, said device
- comprising a heating element (3) and
- comprising an embedding mould (5) for arrangement in the heating element (3),
wherein the embedding mould (5) has a cylindrical cavity (9), which is dimensioned in such a way that an assembly consisting of the catheter (29) with balloon (30) and of the implant (31) mounted on the outer side of the balloon (30) can be arranged in the cavity (9) of the embedding mould (5), wherein a protective film (11) is provided between an inner wall of the cavity (9) of the embedding mould (5) and the assembly, **characterised in that** the protective film (11) is formed as a layer connected to the inner wall of the embedding mould (5).

3. The device according to claim 1, **characterised in that** the cavity (9) of the embedding mould (5) runs parallel to the longitudinal direction of the embedding mould (5) and is formed continuously, wherein the strip-like protective film (11) bears loosely against the inner wall of the embedding mould (5) and can be wound and/or unwound around the assembly and protrudes beyond the continuous cavity (9) of the embedding mould (5) preferably on both sides (21, 22) in the longitudinal direction of the cavity (9).

4. The device according to any one of the preceding claims, **characterised in that** the protective film (11) consists of a plastic material, preferably at least one material of the group comprising PTFE, PFA, PE and LDPE.

5. The device according to any one of the preceding claims, **characterised in that** the embedding mould (5) has a high heat flux.

6. The device according to any one of the preceding claims, **characterised in that** a cooling element (25) is arranged beneath the heating element (5), which cooling element has at least one opening (26) on its upper side for the conduction of a cooling fluid.

7. A method for embedding a balloon (30), arranged on a catheter (29), in an implant (31) having an open-work structure, said method having the following steps:
- producing an assembly consisting of a catheter (29) with balloon (30) and the implant (31) mounted on the outer side of the balloon (30), preferably by crimping the implant (31) onto the balloon (30),
- inserting the assembly into a cavity (9) of an embedding mould (5), which is lined on its inner wall with a protective film (11),
- connecting the catheter (29) to a fluid source,
- heating the embedding mould (5) with assembly in a heating element (3) for a predefined time,
- applying a predefined first internal pressure to the balloon (30) by means of the fluid source connected to the catheter (29),
- cooling the embedding mould (5) with the assembly with a predefined second internal pressure in the balloon (30),
- deflating the balloon (30) after cooling the embedding mould (5) with the assembly,
- removing the assembly from the embedding mould (5).

8. The method according to claim 7, **characterised in that,** prior to the introduction of the assembly into the cavity (9) of the embedding mould (5), a strip-like protective film (11) is wound around the assembly.

9. The method according to either one of claims 7 or 8, **characterised in that,** when introducing the assembly into the embedding mould (5) and/or removing the assembly from the embedding mould (5), the assembly is introduced into the cavity (9) of the embedding mould and/or is removed from the cavity (9) of the embedding mould (5) together with the strip-like protective film (11), wherein the protective film (11) is unwound from the assembly (5) preferably following the removal of the assembly.

10. The method according to any one of claims 7 to 9, **characterised in that** the strip-like protective film (11) is unwound from a roll and is guided through the cavity of the embedding mould.

## Revendications

1. Dispositif d'incorporation d'un ballonnet (30) disposé sur un cathéter (29) dans un implant (31) présentant une structure ajourée,
- avec un élément de chauffage (3) et
- avec un moule d'incorporation (5) à disposer dans l'élément de chauffage (3),
où le moule d'incorporation (5) présente une cavité (9) cylindrique qui est dimensionnée de telle manière qu'un ensemble constitué du cathéter (29) avec le ballonnet (30) et de l'implant (31) monté sur le côté extérieur du ballonnet (30) peut être disposé dans la cavité (9) du moule d'incorporation (5),
où un film de protection (11) est prévu entre une paroi intérieure de la cavité (9) du moule d'incorporation (5) et l'ensemble, **caractérisé en ce que** le film de protection (11) est en forme de bande et que la largeur (B) du film de protection en forme de bande est supérieure au périmètre de la cavité (9) du moule d'incorporation (5).

2. Dispositif d'incorporation d'un ballonnet (30) disposé sur un cathéter (29) dans un implant (31) présentant une structure ajourée,
- avec un élément de chauffage (3) et
- avec un moule d'incorporation (5) à disposer dans l'élément de chauffage (3),
où le moule d'incorporation (5) présente une cavité (9) cylindrique qui est dimensionnée de telle manière qu'un ensemble constitué du cathéter (29) avec le ballonnet (30) et de l'implant (31) monté sur le côté extérieur du ballonnet (30) peut être disposé dans la cavité (9) du moule d'incorporation (5),
où un film de protection (11) est prévu entre une paroi intérieure de la cavité (9) du moule d'incorporation (5) et l'ensemble, **caractérisé en ce que** le film de protection (11) est conçu sous forme d'une couche reliée avec la paroi intérieure du moule d'incorporation (5).

3. Dispositif selon la revendication 1, **caractérisé en ce que** la cavité (9) du moule d'incorporation (5) s'étend parallèlement par rapport à la direction longitudinale du moule d'incorporation (5) et est conçue de part en part, où le film de protection (11) en forme de bande s'applique librement sur la paroi intérieure du moule d'incorporation (5) et peut être enroulée et/ou déroulée autour de l'ensemble et de préférence dépasse, à la fois au niveau des deux côtés (21, 22) dans la direction longitudinale de la cavité (9), par-dessus la cavité (9) allant de part en part du moule d'incorporation (5).

4. Dispositif selon l'une des revendications précédentes, **caractérisé en ce que** le film de protection (11) est constitué d'un matériau synthétique, de préférence, d'au moins un matériau du groupe comprenant le PTFE, le PFA, le PE et le LDPE.

5. Dispositif selon l'une des revendications précédentes, **caractérisé en ce que** le moule d'incorporation (5) possède un flux thermique élevé.

6. Dispositif selon l'une des revendications précédentes, **caractérisé en ce qu'**un élément de refroidissement (25), qui présente au moins un orifice (26) pour le passage d'un fluide de refroidissement sur son côté supérieur, est disposé en dessous de l'élément de chauffage (5).

7. Procédé d'incorporation d'un ballonnet (30) disposé sur un cathéter (29) dans un implant (31) présentant une structure ajourée, avec les étapes suivantes :
- de préparation d'un ensemble constitué du cathéter (29) avec le ballonnet (30) et de l'implant (30) monté sur le côté extérieur du ballonnet (30), de préférence par un sertissage de l'implant (31) sur le ballonnet (30),
- d'insertion de l'ensemble dans une cavité (9) d'un moule d'incorporation (5) qui est revêtue avec un film de protection (1) au niveau de sa paroi intérieure,
- de liaison du cathéter (29) avec une source de fluide,
- de chauffage du moule d'incorporation (5) avec l'ensemble dans un élément de chauffage (3) pour une durée prédéfinie,
- d'application d'une première pression interne prédéfinie au niveau du ballonnet (30) au moyen de la source de fluide reliée avec le cathéter (29),
- de refroidissement du moule d'incorporation (5) avec l'ensemble avec une deuxième pression interne prédéfinie dans le ballonnet (30),
- après le refroidissement du moule d'incorporation (5) et la déflation du ballonnet (30),
- le prélèvement de l'ensemble à partir du moule d'incorporation (5).

8. Procédé selon la revendication 7, **caractérisé en ce qu'**avant l'insertion de l'ensemble dans la cavité (9) du moule d'incorporation (5), un film de protection (11) en forme de bande est enroulé autour de l'ensemble.

9. Procédé selon l'une des revendications 7 à 8, **caractérisé en ce que** lors de l'insertion de l'ensemble dans le moule d'incorporation (5), et/ou lors du prélèvement de l'ensemble hors du moule d'incorporation (5), l'ensemble est mené conjointement avec le film de protection (11) en forme de bande à l'intérieur de la cavité (9) du moule d'incorporation, respectivement est mené en dehors de la cavité (9) du moule d'incorporation (5), où, de préférence, le film de protection (11) est ensuite déroulé de l'ensemble (5) après le prélèvement de l'ensemble.

10. Procédé selon l'une des revendications 7 à 9, **caractérisé en ce que** le film de projection (11) en forme de bande est déroulé à partir d'un rouleau et est mené à travers la cavité du moule d'incorporation.
